# FASCICULE DE BREVET EUROPEEN

(11) **EP 2 266 503 B1**
(45) Date de publication et mention de la délivrance du brevet: **04.01.2017**
(21) Numéro de dépôt: 10012198.7
(22) Date de dépôt: 02.08.2002
(51) Int. Cl.: A61F 2/24, A61F 2/06

(54) **DISPOSITIF D'IMPLANTATION D'UNE PROTHÈSE DE VALVE CARDIAQUE**
VERFAHREN ZUR HERSTELLUNG FÜR EINE HERZKLAPPENPROTHESE
DEVICE TO IMPLANT A CARDIAC VALVE PROSTHESIS

(30) Priorité: 03.08.2001 FR 0110444
(43) Date de publication de la demande: 29.12.2010
(62) Demande divisionnaire de: 02291953.4
(73) Titulaire: JenaValve Technology, Inc., Irvine, CA 92618 (US); Bonhoeffer, Philipp, London NW1 9XP (GB)
(72) Inventeur: Bonhoeffer, Philipp, London NW19XP (GB); Younes, Boudjemline, 94000 Creteil (FR)
(74) Mandataire: Zwicker, Jörk

(56) Documents cités:
- WO-A-00/69367
- WO-A-99/42058
- WO-A2-01/49213
- US-A- 5 053 008

## Description

### Domaine technique

La présente invention se rapporte à un dispositif d'implantation d'un implant ainsi qu'à un procédé de mise en place du dispositif dans un élément tubulaire.

Le problème à l'origine de l'invention concernait l'implantation de valves cardiaques. Encore récemment, cela nécessitait une intervention chirurgicale à coeur ouvert, avec des étapes telles que l'arrêt du coeur, la mise en oeuvre d'une circulation sanguine extra-corporelle et le redémarrage du coeur après l'implantation de valves cardiaques de remplacement. Ces opérations chirurgicales sont lourdes et délicates et elles présentent des risques mortels liés aux chocs opératoires.

### Art antérieur

Le document US 5 824 063 décrit ainsi un dispositif portant des valves cardiaques de remplacement, dispositif comprenant un implant tubulaire en matériau synthétique portant intérieurement une valve de remplacement en matériau naturel.

Les documents US 5 855 601 et US 5 868 783 décrivent de nouvelles méthodes d'implantation de valves cardiaques, qui présentent l'avantage d'éviter une chirurgie à coeur ouvert. Ces méthodes prévoient l'implantation, par cheminement dans le système de circulation sanguine, d'un dispositif à valve de remplacement cardiaque comprenant un cylindre intravasculaire expansible radialement portant intérieurement une valve biologique. Une partie gonflable d'un cathéter ballon est placée à l'intérieur du cylindre porteur et l'implantation est réalisée par introduction dans une veine et translation jusqu'à la valve défaillante au moyen d'un cathéter. Une visualisation sur écran d'imagerie bidimensionnelle permet de détecter que le cylindre porteur a atteint la position voulue et le cylindre est alors dilaté par gonflage du ballonnet à travers le cathéter et il conserve sa forme expansée. Le ballonnet est alors dégonflé et retiré avec le cathéter. Le cylindre porteur présente une enveloppe étanche qui est ainsi plaquée contre la paroi de l'artère, afin d'éviter un contournement de la valve de remplacement par le flux sanguin.

WO01/49213 décrit une prothèse de valve cardiaque et un cathéter pour sa délivrance.

5 Toutefois, lorsqu'il s'agit de l'aorte, ce procédé n'est pas applicable car les artères coronaires débouchent à proximité des valves natives défaillantes, si bien que le cylindre porteur est susceptible de les obturer, provoquant la mort du patient.

### But de l'invention

Les inventeurs de la présente demande ont alors pensé à ménager deux ouvertures correspondantes dans la paroi de l'enveloppe du cylindre porteur. Cependant, pour que ces deux ouvertures soient placées en face des deux coronaires, il faut totalement maîtriser le positionnement du cylindre porteur dans l'aorte. Un suivi sur l'écran permettrait bien de vérifier l'avance, ou positionnement axial, du cylindre porteur, mais sa position angulaire ne serait ni visible, ni maîtrisée.

Les demandeurs ont alors trouvé une solution, exposée plus loin, permettant de maîtriser le positionnement du cylindre porteur.

Ils ont alors songé à l'appliquer à la résolution du problème plus général de positionnement d'un dispositif ou véhicule de transport d'un implant dans un élément tubulaire difficile d'accès et pour lequel une imagerie est insuffisante ou même impossible. Le champ d'application peut ainsi concerner des domaines autres que le médical, par exemple l'industrie pétrolière ou nucléaire, pour implanter des capteurs, vannes ou autres.

La portée de la présente demande ne doit donc pas être considérée comme devant être limitée à la résolution du problème d'origine. D'une façon plus générale, l'invention vise à permettre la mise, à un endroit, difficile d'accès, dans un élément tubulaire, d'un dispositif prévu pour porter un implant, quelle que soit la fonction de l'implant.

### Résumé de l'invention

A cet effet, l'invention est décrite dans la revendication 1.

Ainsi, on peut faire avancer de façon aveugle le dispositif et ses moyens palpeurs permettent de détecter automatiquement la cavité et de s'y positionner.

On peut ainsi accéder à la position finale voulue, même à travers un rétrécissement de l'élément tubulaire, par exemple une artère d'accès à une artère de plus grand diamètre.

Il est aussi décrit à titre d'exemple un procédé, non chirurgical et à but non thérapeutique, d'implantation du dispositif de l'invention, à une position déterminée dans un élément tubulaire présentant une paroi comportant une cavité, procédé caractérisé par le fait que
- un utilisateur introduit le dispositif par une extrémité ouverte de l'élément tubulaire,
- il actionne les moyens d'entraînement pour faire avancer le dispositif jusqu'à une position en amont de la position déterminée,
- il commande les moyens de téléactivation des moyens palpeurs et, l'avance se poursuivant,
- il arrête l'actionnement des moyens d'entraînement lorsqu'il détecte un blocage de l'avance, traduisant le fait que les moyens palpeurs se sont positionnés dans la cavité.

### Brève description des dessins

Les caractéristiques et avantages de la présente invention apparaîtront plus clairement à l'aide de la description suivante d'une forme particulière de réalisation du dispositif de l'invention et d'une variante, ainsi que du procédé de mise en oeuvre de celui-ci, en référence au dessin annexé, dans lequel :
- la figure 1 est une vue en section latérale du dispositif de l'invention, représentant des éléments palpeurs de positionnement et d'ancrage, associés à un cylindre porteur d'une prothèse de valve, le tout recouvert de deux gaines d'activation amovibles concentriques,
- la figure 2 correspond à la figure 1, les éléments palpeurs de positionnement et d'ancrage ayant été déployés radialement par recul axial de la gaine externe,
- la figure 3 correspond aux figures 1 et 2, le cylindre porteur entouré des éléments palpeurs de positionnement et d'ancrage ayant été déployé radialement après recul axial de la gaine interne,
- la figure 4 est une vue latérale du cylindre porteur et des éléments palpeurs de positionnement et d'ancrage,
- la figure 5 est une vue latérale en perspective des éléments palpeurs de positionnement et d'ancrage,
- la figure 6 est une vue de face schématique du dispositif de l'invention, et
- la figure 7 est une coupe latérale schématique de la variante.

### Description détaillée

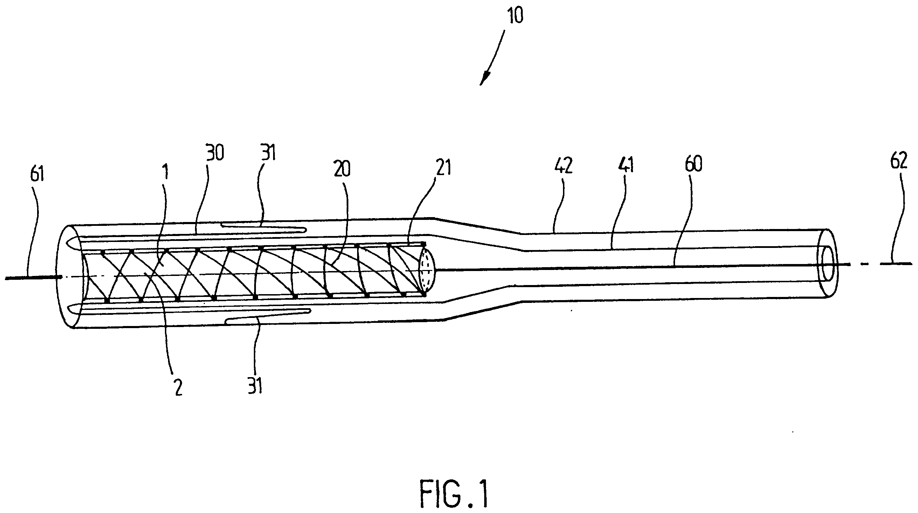

Comme le montre la figure 1, le présent exemple de réalisation correspond au problème médical exposé au début, d'implantation d'une valve de remplacement fonctionnel de la valve native aortique. Le dispositif 10 d'implantation de valves comporte un élément porteur 20 de réception d'implant, solidaire d'une pluralité d'éléments ou doigts palpeurs 30, 31, ici régulièrement répartis angulairement tout autour, de positionnement et d'ancrage par rapport à un relief, précisément une cavité de la paroi aortique, le dispositif 10 étant relié de façon amovible à un cathéter 60 de mise en place. Le dispositif 10 est associé à deux gaines concentriques 41, 42 de commande de téléactivations successives, par expansion radiale, des éléments palpeurs 30, 31 puis de l'élément porteur 20. La direction d'avance du dispositif 10 est donc vers la gauche sur les figures 1 à 3. La référence 62 représente un axe de symétrie et de direction d'entraînement du dispositif 10 et du cathéter 60.

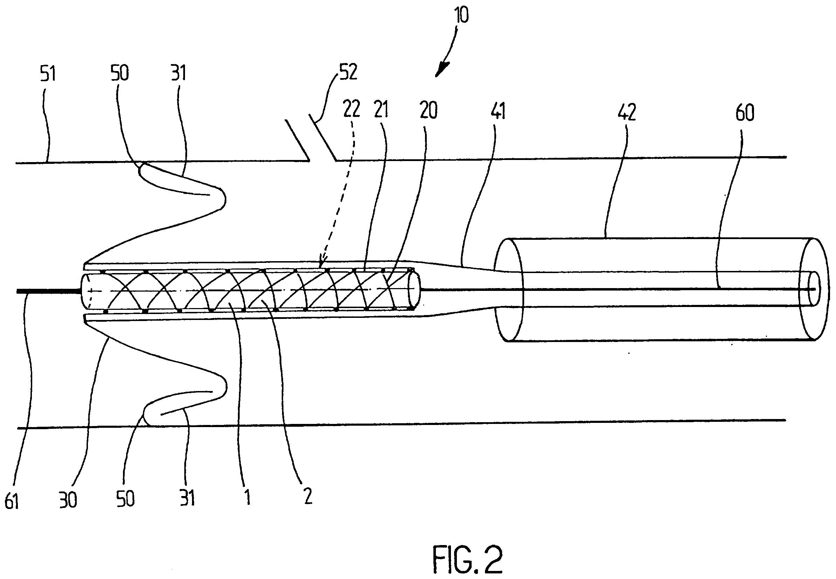

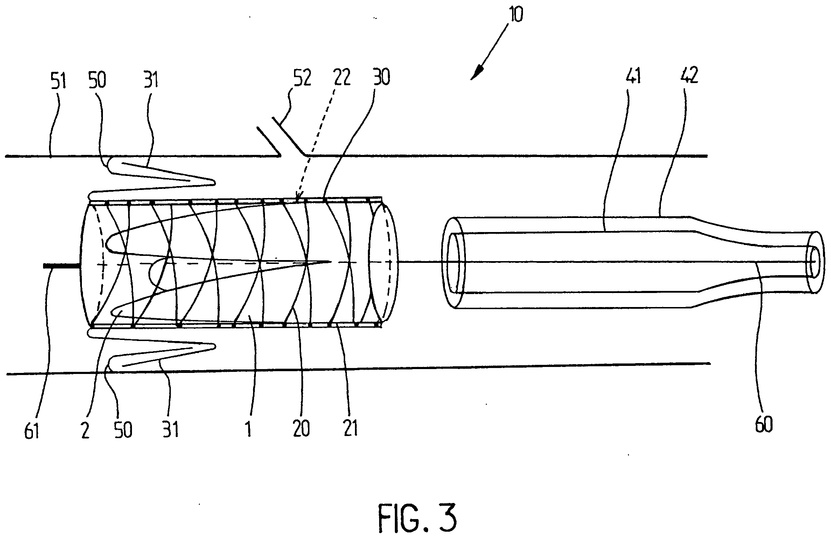

La valve d'implantation forme une prothèse 1 constituée de valvules 2 de la valve dont la forme et la taille correspondent parfaitement, en position fonctionnelle, à celles de valves natives aortiques 50 (fig. 2). La prothèse 1 est fixée à l'élément véhicule porteur 20 de réception d'implant, ici constitué par un treillis cylindrique en un matériau bio-compatible tel que l'acier, des alliages d'or et, de préférence comme ici, le nitinol, constitué d'un alliage nickel-titane à mémoire de forme présentant la faculté de reprendre sa forme après avoir été initialement déformé, ici par compression radiale. La fixation de la prothèse 1 au treillis cylindrique 20 en nitinol est effectuée en des endroits bien définis laissant libres des régions, correspondant aux valvules 2 après leur déploiement tel qu'illustré plus loin en regard de la figure 3, à partir de la position reployée de la figure 2.

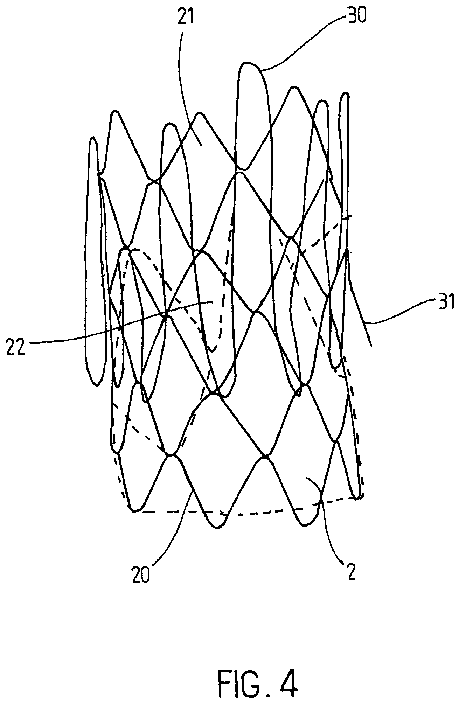

La figure 4 représente le treillis cylindrique 20 dans sa forme déployée, portant intérieurement les valvules 2 aussi déployées, sur lequel sont raccordés les éléments palpeurs 30, 31, ici sous la forme d'une couronne extérieure globalement cylindrique de boucles de fil dont l'une (31) au moins, ici en fait trois, fait saillie latéralement et vers l'avant, à l'opposé du cathéter 60. Dans cet exemple, les boucles 31 s'étendent, en position déployée, dans une direction inclinée d'environ 30 degrés vers l'avant (direction d'avance vers la position visée) par rapport à l'axe 62 du treillis 20 et de la couronne 30. Les éléments palpeurs 30, 31 sont solidarisés au treillis cylindrique 20 de telle manière que leurs positions axiales et angulaires par rapport à celui-ci sont parfaitement définies. L'ensemble, treillis cylindrique 20 et éléments palpeurs 30, 31, est ici constitué en le matériau bio-compatible auto expansible susmentionné.

Le treillis porteur cylindrique 20 est ici recouvert d'une enveloppe latérale étanche 21 destinée à être plaquée sur la paroi aortique pour éviter un contournement de la circulation sanguine.

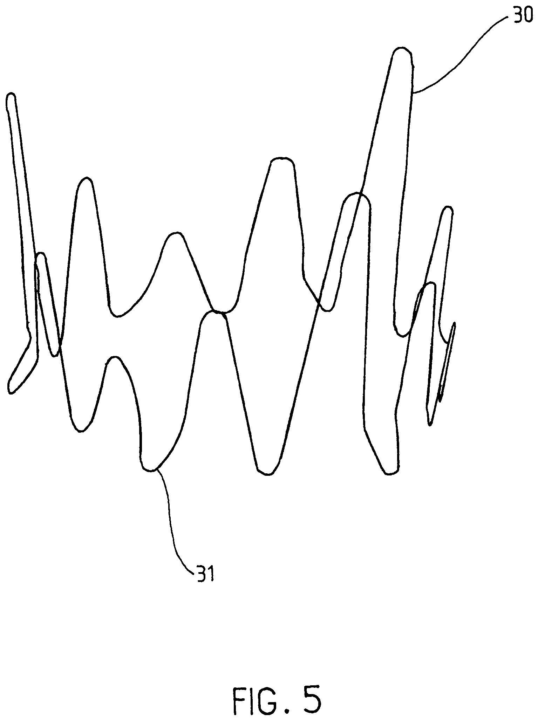

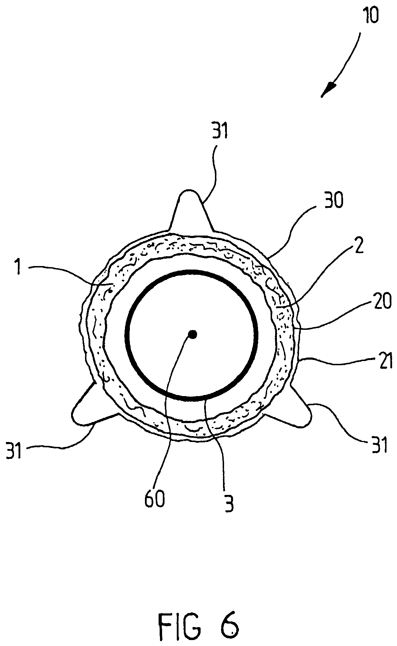

La figure 5 représente en perspective les éléments palpeurs 30, 31. La figure 6 est une vue schématique, selon une direction axiale du dispositif 10, montrant les trois boucles 31 faisant saillie latéralement à l'extérieur du grillage tubulaire 20 qui les porte, alors que les valvules 2 de la valve à implanter sont fixées intérieurement au cylindre porteur 20.

En outre, si nécessaire, un ballonnet 3 gonflable, solidaire du cathéter 60, peut être ici placé à l'intérieur du cylindre porteur 20, pour être alimenté en liquide sous pression à travers un conduit du cathéter 60 afin de provoquer ou d'aider l'expansion radiale du cylindre porteur 20 jusqu'à la forme déployée voulue.

Comme les éléments palpeurs 30, 31 sont fabriqués en un matériau autoexpansible tel que le nitinol, ou un élément équivalent formant patte ou doigt de saillie élastique, le dispositif 10 est recouvert d'une gaine d'inhibition 42 pour maintenir les éléments palpeurs 30, 31 en une position reployée, les boucles 31 étant rabattues sur la couronne 30 et donc aussi sur le treillis 20. La gaine 42 se prolonge pour recouvrir tout le cathéter 60. Une deuxième gaine 41, sensiblement de même longueur et sans effet sur les éléments palpeurs 30, 31, est ici de même prévue pour maintenir le cylindre porteur 20 en position reployée, afin d'éviter un déploiement inopiné même en l'absence de gonflage du ballonnet 3. Les deux gaines 41, 42, sont montées concentriquement sur le cathéter 60. Les gaines 41 et 42 sont accessibles depuis l'extrémité du cathéter 60 opposée au dispositif 10. Les éléments 3, 41, 42 et 60 constituent un ensemble cathéter fonctionnel séparable du dispositif 10, pour le positionnement et la mise en service de ce dernier et de sa charge utile (2).

Les deux gaines 41, 42 inhibent le déploiement radial de la structure 20, 30, 31 jusqu'à ce que celle-ci atteigne la région de la valve native aortique 50 à remplacer fonctionnellement, et permettent ainsi l'introduction du dispositif 10 dans le système de circulation sanguine, telle qu'une artère incisée de diamètre réduit. Comme indiqué, le cathéter 60, avec le ballonnet 3, est solidarisé de façon amovible au dispositif d'implantation 10 afin de permettre une avance axiale du dispositif d'implantation 10 dans le système de circulation sanguine jusqu'à l'endroit de l'implantation, et le retrait de l'ensemble cathéter 3, 41, 42, 60.

Pour sa libération, le cathéter 60 comporte dans cet exemple, à l'extrémité fixée au cylindre porteur 20, une pince à effet ressort (non représentée), avec des crocs télécommandés, montés rotatifs radialement, de solidarisation au dispositif 10 et il comporte un fil métallique central coulissant de télécommande pour repousser axialement les branches ou crocs de la pince afin de les écarter radialement et ainsi libérer le cathéter 60 du dispositif 10 d'implantation selon le principe d'une pince à sucre.

Lorsque le treillis cylindrique 20 est déployé, la pression sur la paroi interne aortique est assurée par l'effet de la mémoire de forme qui assure ainsi la dilatation radiale de la prothèse 1. La valvule native défaillante 50 est aplatie en étant plaquée par le grillage tubulaire 20 contre la paroi interne de l'aorte, chacune des trois boucles 31 faisant saillie latéralement ayant préalablement été engagée dans une, particulière, des trois valvules natives 50 et étant de même plaquée pour confirmer son ancrage. Les valvules 50 sont ainsi pincées entre le treillis 20, 30 et les boucles respectives 31.

Le procédé d'implantation du dispositif 10 décrit ci-dessus, selon le mode préféré de mise en oeuvre, comporte les étapes suivantes. Après introduction du dispositif 10 d'implantation dans le système circulatoire, et après l'avoir poussé au moyen du cathéter 60 jusqu'à une position en amont de la position finale visée, précisément ici lorsque le dispositif 10 est arrivé dans l'aorte, et qu'un espace de plus grand diamètre lui est ainsi offert, l'étape suivante consiste à libérer les boucles latérales 31, initialement plaquées sur le treillis 20, 30 reployé. La libération des boucles 31 s'effectue par retrait de la gaine externe d'inhibition 42 (figure 2), c'est-à-dire recul, en maintenant la poussée sur le cathéter 60. L'avance du dispositif 10 se poursuivant, les boucles 31, faisant alors nettement saillie latéralement vers l'avant par rapport à la direction axiale d'avance, à l'opposé du cathéter 60, elles constituent une sorte de trépied et pénètrent simultanément dans les trois valvules natives 50 respectives, sensiblement identiques, constituant une rangée en anneau complet de poches jointives, s'étendant chacune sur 120 degrés, garnissant au total la totalité du pourtour de la paroi interne de l'aorte 51. Chaque valvule native 50 présente un fond arrondi.

Chaque saillie latérale 31, tournée vers l'avant, bute sur le fond de la valvule 50 native en regard, en général en un point quelconque à distance du point le plus "bas" de ce fond, c'est-à-dire le plus éloigné du cathéter 60. Il s'agit alors d'une butée partielle car l'avance axiale du dispositif 10 se poursuit par poussée du cathéter 60, la poussée axiale sur le dispositif 10 provoquant son glissement vers le point le plus bas. Le fond de la valvule 50 constitue ainsi une sorte de piste de guidage ou plan incliné (non orthogonal à l'axe (62) de l'aorte) qui, par réaction à la force d'avance axiale, engendre une force de réaction circonférentielle provoquant la rotation du dispositif 10 jusqu'à ce que la boucle de palpeur considérée 31 atteigne le point le plus bas, qui correspond à une butée totale (à plan tangent orthogonal à l'axe (62) de l'aorte 51), donc correspond à la position finale recherchée, axiale et angulaire, du dispositif 10.

Chaque saillie latérale 31, à extrémité arrondie, ici en boucle, pour pouvoir glisser sur le fond de la valvule 50, constitue ainsi, par coopération avec le fond arrondi des valvules natives 50 de profondeur variable de façon continue, un moyen d'entraînement en rotation des éléments palpeurs 30, 31 et donc aussi du treillis cylindrique 20, qui en est solidaire. Toutefois, si par hasard les saillies latérales 31 butaient sur une commissure des valvules natives 50, le dispositif 10 d'implantation pourrait être légèrement reculé et l'opérateur tordrait le cathéter 60 pour qu'il pivote angulairement afin de recommencer l'opération de positionnement et d'ancrage.

L'ensemble, éléments palpeurs 30, 31 et treillis cylindrique 20, étant ainsi positionné axialement et angulairement par rapport au relief particulier de l'aorte que constituent les valvules natives 50, il se trouve donc automatiquement positionné par rapport aux deux orifices coronariens (52) dont la position axiale et angulaire par rapport aux valvules 50 est déterminée et connue, la distance axiale valvules-coronaires dépendant évidemment de la taille du patient.

Dans le cas ici considéré dans lequel les trois valvules natives 50 forment un pourtour circulaire de la paroi aortique s'étendant sur 360 degrés, une seule saillie latérale 31 est suffisante pour le positionnement modulo 120 degrés et l'ancrage du treillis cylindrique 20. Comme évoqué plus haut, dans un cas général, il pourrait n'y avoir qu'un seul palpeur 30, 31 coopérant avec une rangée de cavités ou poches garnissant tout le pourtour de l'élément tubulaire, ou bien encore une seule poche ou cavité 50 n'occupant qu'un secteur du pourtour et une pluralité de palpeurs 30, 31 tout autour du dispositif 10 pour que l'un d'entre eux coopère avec la cavité.

On notera que, dans le présent exemple, on peut tolérer un positionnement modulo 120 degrés car les deux coronaires (52) présentent naturellement sensiblement cet angle. Si ce n'était pas le cas, il faudrait élargir latéralement deux ouvertures ou échancrures 22 prévues dans l'enveloppe 21 pour qu'elles soient positionnées en face des coronaires (52) (fig. 4 et position repérée sur la fig. 3), ou encore palper, par les palpeurs 31, les coronaires (52) elles-mêmes, qui constituent aussi des cavités de l'aorte 51, et non plus palper les valvules natives 50. Ce cas correspond à la variante exposée plus loin.

Le positionnement étant ainsi effectué, l'étape suivante, telle que visualisée à la figure 3, consiste à déployer le treillis cylindrique 20 portant intérieurement les valvules 2 par retrait de la gaine interne 41 de maintien, pour consolider l'ancrage et faire passer les valvules 2 à leur forme fonctionnelle. Pour la clarté du dessin, en particulier des saillies 31, le treillis 20 a été représenté à un diamètre relatif réduit, alors qu'en fait il correspond à celui de l'aorte 51, avec un léger supplément pour assurer la pression latérale voulue. De même, on a représenté deux saillies 31, alors qu'en fait elles sont écartées de 120 degrés, le plan de la figure 3 n'en coupant en réalité qu'une seule. Pour cette raison, il n'a été dessiné qu'une seule coronaire (52).

Les trois boucles 31 faisant saillie assurent toutefois par elles-mêmes un ancrage élémentaire dans le fond des poches que constituent les valvules natives 50 et elles garantissent ainsi une stabilité de position de la prothèse 1. Après quelques semaines, un tissu fibreux viendra recouvrir la prothèse 1, coopérant avec les saillies latérales 31 pour améliorer encore sa fixation.

On notera toutefois que, en position déployée des éléments palpeurs 31, il n'est pas nécessaire que les extrémités libres de ceux-ci soient en appui ferme sur la paroi de l'aorte 51. Il suffit que leur extension radiale soit suffisante pour qu'elles s'accrochent, au passage, aux valvules 50. De ce fait, lorsque le déploiement des éléments palpeurs 31 s'effectue, en amont de la position finale, la translation axiale ultérieure du dispositif 10, jusqu'à cette position, s'effectue sans frottement "dur", sous pression, de la part des boucles 31 sur la paroi de l'aorte 51. Celle-ci ne court ainsi aucun risque d'endommagement par éraflure ou perçage, les boucles 31 étant des palpeurs, qui suivent la paroi de l'aorte 51 pour détecter les valvules 50. Comme évoqué plus haut, des pattes ou languettes à bout arrondi peuvent aussi convenir.

Les boucles de palpeur 31 n'ont donc pas ici pour fonction principale d'ancrer de façon très ferme le dispositif 10 dans l'aorte 51, puisqu'elles ne visent pas à excercer une forte presson radiale d'ancrage. Comme indiqué plus haut, il ne s'agit que d'un ancrage élémentaire. C'est ensuite le déploiement radial du treillis 20 qui engendre, par la mémoire de forme, une pression radiale d'ancrage définitif qui plaque sous pression le treillis 20 contre la paroi de l'aorte 51 et bloque ainsi tout déplacement relatif, tel que recul du dispositif 10 qui serait dû au flux sanguin, de direction opposée à celle de l'introduction du dispositif 10. Les éléments palpeurs 31 sont alors fonctionnellement superflus. Ils contribuent toutefois au maintien en position par le pincement des valvules 2. Comme le treillis 20 présente une surface de contact avec l'aorte 51 relativement élevée, tout risque d'endommagement de celle-ci est exclu. Le matériau à mémoire de forme permet de déterminer précisément la pression radiale exercée sur l'aorte 51, le diamètre ainsi accru de celle-ci étant donc parfaitement défini, ce qui élimine tout risque de contrainte radiale excessive.

Le procédé de l'invention peut être mis en oeuvre de façon non chirurgicale et dans un but non thérapeutique, pour implanter le dispositif 10 (ou équivalent) à une position déterminée dans un élément tubulaire présentant une paroi comportant une cavité, le procédé comportant les étapes suivantes :
un utilisateur introduit le dispositif (10) par une extrémité ouverte de l'élément tubulaire,
l'utilisateur actionne les moyens d'entraînement (60) (cathéter, aimant externe ou autre) pour faire avancer le dispositif (10) jusqu'à une position en amont de la position déterminée,
il commande les moyens d'activation (42) des moyens palpeurs (30, 31) et, l'avance se poursuivant,
il arrête l'actionnement des moyens d'entraînement (60) lorsqu'il détecte un blocage de l'avance, traduisant le fait que les moyens palpeurs (30, 31) se sont positionnés dans la cavité.

Pour faciliter l'entraînement du dispositif 10, celui-ci peut être associé à une sorte de rostre 61 précurseur (fig. 1 à 3) formant guide, sous forme d'élément cylindrique de diamètre limité, solidaire du cathéter 60.

On notera que le dispositif d'implantation selon l'invention peut d'abord être implanté seul, sans implant ou charge utile, ce dernier pouvant être implanté dessus par la suite selon le même principe. En pareil cas, le dispositif de l'invention comporte des moyens de réception du deuxième support, à venir, de l'implant, agencés pour assurer le positionnement et l'ancrage du deuxième support à la fois axialement, par butée, et radialement, avec des moyens de détrompage angulaire tels que doigt ou cavité prévu pour coopérer avec un élément de forme complémentaire du deuxième support.

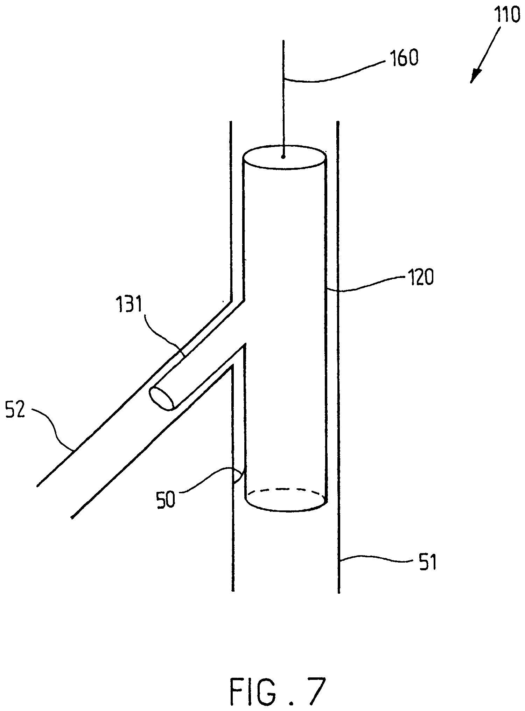

Dans la variante selon la figure 7, le dispositif d'implantation porte la référence 110 et comporte les éléments fonctionnels homologues de ceux du dispositif 10, avec la même référence précédée de la centaine 1, qui n'ont toutefois pas tous été représentés, dans un but de clarté. L'élément porteur cylindrique 120 est solidaire d'un élément palpeur 131 qui en fait saillie latéralement et qui présente une constitution de même genre que celle de l'élément porteur 120. Précisément, l'élément palpeur 131 se présente sous la forme d'un cylindre, reployé radialement au repos. Lorsque le dispositif 110 est poussé par le cathéter 160, vers le bas sur la figure 7, depuis une position en amont de celle représentée, il s'engage dans la coronaire 52 lorsque son extrémité libre est ainsi libérée du contact avec la paroi interne de l'aorte 51.

Le dispositif 110 constitue ainsi une sorte de fourche qui se bloque par butée dans la bifurcation entre l'aorte 51 et la coronaire 52. Lorsque la position de butée est atteinte, les deux éléments cylindriques 120, 131 sont déployés par deux ballonnets respectifs et forment une sorte de gant à deux doigts.

Ainsi, lors de la phase de mise en place, le palpeur 131 présente une forme reployée radialement, donc à diamètre réduit ne risquant pas d'obturer la coronaire 52. Ensuite, le palpeur 131 est déployé, par gonflage du ballonnet associé de téléactivation, et constitue un doublage, ou "enveloppe" interne, plaqué contre la paroi interne de la coronaire 52 selon le principe exposé précédemment pour le cylindre porteur 20.

On remarque que, comme les éléments 120 et 131 occupent chacun une branche particulière 51, 52, ils peuvent être considérés comme fonctionnellement homologues, avec éventuellement les deux fonctions principales. Chacun d'eux peut en effet être le porteur d'une charge utile (2) et peut aussi être considéré comme étant un palpeur, puisque l'aorte 51 peut être considérée (fonctionnellement dans le cadre de la présente invention) comme étant une cavité ou embranchement par rapport à la coronaire 52. Ainsi, les moyens palpeurs comportent un élément cylindrique 131 agencé pour passer d'une forme reployée à une forme déployée radialement, d'appui sur une paroi de la cavité, ici la coronaire 52, sous l'effet de moyens de téléactivation (ballonnet et cathéter 160).

Pour éviter les aléas de passage en position de couplage du palpeur 131 à la coronaire 52, dus à un décalage angulaire éventuel, qui nécessiterait plusieurs tentatives, il peut être prévu de passer préalablement un fil guide dans la coronaire 52 et la partie amont de l'aorte 51, le dispositif 110 étant enfilé dessus à travers le palpeur 131 qui est ainsi orienté angulairement vers la coronaire 52. Un autre fil guide peut de même guider le cylindre 120 dans l'aorte 51.

## Revendications

1. Dispositif (10) d'implantation d'une valve comprenant :
(a) un élément porteur (20) configuré pour supporter des valvules artificielles (2) et configuré pour être déployable d'une configuration reployée à une configuration déployée ; et
(b) un ensemble cathéter (3, 41, 42, 60) configuré pour maintenir le dispositif (10) d'implantation dans une configuration reployée pendant l'acheminement jusqu'à une position en amont de la position finale visée ;
**caractérisé en ce que** le dispositif (10) d'implantation comprend en outre :
(c) au moins un élément palpeur (30, 31) s'étendant de l'élément porteur, l'élément porteur (20) et les éléments palpeur (30, 31) être disposés de telle sorte que des valvules naturelles (50) peuvent être pincées entre l'élément porteur (20) et les éléments palpeur (30, 31) ;
tandis que ledit au moins un élément palpeur (30, 31) est déformable et agencé pour passer d'une forme reployée à une forme déployée fonctionnelle pour détecter une cavité (50, 52) et s'y positionner en référence à la position de celle-ci.

2. Dispositif (10) d'implantation selon la revendication 1,
dans lequel le dispositif (10) d'implantation est formé d'un matériau à mémoire de forme ; et
dans lequel le dispositif (10) d'implantation est configuré à être libéré successivement de l'ensemble cathéter (3, 41, 42, 60) de telle sorte qu'au moins un élément palpeur (30, 31) est libéré à une configuration permettant à être reçu dans une cavité (50, 52) associée avec une valvule naturelle (50) au lieu distant, tandis que un reste du dispositif (10) d'implantation est maintenu dans la configuration reployée par l'ensemble cathéter (3, 41, 42, 60).

3. Dispositif (10) d'implantation selon la revendication 1 ou 2, dans lequel le dispositif (10) d'implantation peut être libéré complètement, dans une étape suivante, de l'ensemble cathéter (3, 41, 42, 60).

4. Dispositif (10) d'implantation selon l'une des revendications 1 à 3, dans lequel les éléments palpeur sont à base d'un matériau à mémoire de forme.

5. Dispositif (10) d'implantation selon l'une des revendications 1 à 4, dans lequel l'ensemble cathéter (3, 41, 42, 60) est amovible et comporte une gaine d'inhibition (42) pour maintenir les éléments palpeurs (30, 31) en position reployée, gaine s'étendant au-delà des éléments palpeurs (30, 31) pour les libérer par recul relatif de la gaine par rapport à un cathéter (60) d'entraînement du dispositif par poussée.

6. Dispositif (10) d'implantation selon l'une des revendications 1 à 5, dans lequel les éléments palpeurs (30, 31) comportent une couronne (30), de forme globalement cylindrique, de boucles (31) de fil à rigidité limitée en direction radiale, l'une au moins des boucles (31) étant prévue pour faire saillie latéralement afin de constituer un élément palpeur.

7. Dispositif (10) d'implantation selon l'une des revendications 1 à 6, dans lequel les éléments palpeurs (30, 31) comportent une pluralité de doigts palpeurs (31) régulièrement répartis angulairement et agencés pour, en forme déployée, s'étendre selon des directions respectives inclinées, à angle aigu, sur un axe longitudinal (62) d'entraînement en avant du dispositif (10) vers la cavité (50, 52).

8. Dispositif (10) d'implantation selon l'une des revendications 1 à 7, dans lequel les éléments palpeurs (30, 31) sont solidaires de moyens déformables (20) de réception d'un implant (2) agencés pour, sous l'action de moyens de libération (41), passer d'une forme reployée à une forme déployée radialement, d'appui sur la paroi de l'élément tubulaire (51) et de mise en service de l'implant (2).

9. Dispositif (10) d'implantation selon l'une des revendications 1 à 8, dans lequel les moyens (20) de réception de l'implant (2) comportent un treillis de forme globalement cylindrique, pour porter l'implant, présentant une rigidité limitée en direction radiale, et les moyens de libération comportent une gaine amovible (41) de maintien du treillis porteur (20) en forme reployée, s'étendant axialement au-delà du treillis pour le libérer par recul relatif de la gaine (42) par rapport à un cathéter (60) d'entraînement du dispositif par poussée, les gaines (41, 42), d'inhibition et de maintien, étant concentriques.

10. Dispositif (10) d'implantation selon l'une des revendications 1 à 9, dans lequel les moyens de réception (20) d'un implant sont recouverts d'une enveloppe latérale (21) d'étanchéité, destinée à être plaquée sur la paroi de l'élément tubulaire (51) par lesdits moyens de réception (20) d'un implant, et l'enveloppe (21) comporte au moins une ouverture ou échancrure (22) occupant une position angulaire déterminée par rapport aux éléments palpeurs (30, 31).

11. Dispositif (10) d'implantation selon l'une des revendications 1 à 10, dans lequel les moyens palpeurs (131) comportent un élément cylindrique agencé pour passer d'une forme reployée à une forme déployée radialement, d'appui sur une paroi de la cavité (52) sous l'effet de moyens de téléactivation.

12. Dispositif (10) d'implantation selon l'une des revendications 1 à 11, dans lequel le dispositif comprend un, deux ou trois éléments palpeur (30, 31).

## Patentansprüche

1. Vorrichtung (10) zum Implantieren einer Klappe, umfassend:
(a) ein Tragelement (20), das dazu ausgelegt ist, künstliche Herzklappen (2) zu tragen, und das dazu ausgelegt ist, aus einer eingefahrenen Konfiguration in eine ausgefahrene Konfiguration ausfahrbar zu sein; und
(b) eine Katheteranordnung (3, 41, 42, 60), die dazu ausgelegt ist, die Implantationsvorrichtung (10) während des Einführens bis zu einer Position vor der beabsichtigten Endposition in einer eingefahrenen Konfiguration zu halten;
**dadurch gekennzeichnet, dass** die Implantationsvorrichtung (10) ferner Folgendes umfasst:
(c) wenigstens ein Fühlerelement (30, 31), das sich vom Tragelement erstreckt, wobei das Tragelement (20) und die Fühlerelemente (30, 31) derart angeordnet sind, dass die natürlichen Herzklappen (50) zwischen dem Tragelement (20) und den Fühlerelementen (30, 31) eingeklemmt werden können;
wobei das wenigstens eine Fühlerelement (30, 31) verformbar und dazu vorgesehen ist, von einer eingefahrenen Form in eine ausgefahrene Funktionsform überzugehen, um einen Hohlraum (50, 52) zu erfassen und sich darin mit Bezug auf dessen Position zu positionieren.

2. Implantationsvorrichtung (10) nach Anspruch 1,
wobei die Implantationsvorrichtung (10) aus einem Formgedächtnismaterial besteht; und wobei die Implantationsvorrichtung (10) so ausgelegt ist, dass sie nach und nach aus der Katheteranordnung (3, 41, 42, 60) derart freigegeben wird, dass wenigstens ein Fühlerelement (30, 31) in einer Konfiguration freigegeben wird, die es ermöglicht, in einem Hohlraum (50, 52) aufgenommen zu werden, der mit einer natürlichen Herzklappe (50) an einem entfernten Ort assoziiert ist, während ein Rest der Implantationsvorrichtung (10) durch die Katheteranordnung (3, 41, 42, 60) in der eingefahrenen Konfiguration gehalten wird.

3. Implantationsvorrichtung (10) nach Anspruch 1 oder 2, wobei die Implantationsvorrichtung (10) in einem Folgeschritt vollständig aus der Katheteranordnung (3, 41, 42, 60) freigegeben werden kann.

4. Implantationsvorrichtung (10) nach einem der Ansprüche 1 bis 3, wobei die Fühlerelemente aus einem Formgedächtnismaterial bestehen.

5. Implantationsvorrichtung (10) nach einem der Ansprüche 1 bis 4, wobei die Katheteranordnung (3, 41, 42, 60) abnehmbar ist und eine Hemmhülle (42) aufweist, um die Fühlerelemente (30, 31) in eingefahrener Stellung zu halten, wobei die Hülle sich über die Fühlerelemente (30, 31) hinaus erstreckt, um diese durch relatives Zurücksetzen der Hülle bezüglich eines Katheters (60) zum Verschieben der Vorrichtung freizugeben.

6. Implantationsvorrichtung (10) nach einem der Ansprüche 1 bis 5, wobei die Fühlerelemente (30, 31) eine insgesamt zylinderförmige Krone (30) aus Drahtschlingen (31) mit begrenzter Steifigkeit in radialer Richtung aufweisen, wobei wenigstens eine der Schlingen (31) dazu vorgesehen ist, seitlich abzustehen, um ein Fühlerelement zu bilden.

7. Implantationsvorrichtung (10) nach einem der Ansprüche 1 bis 6, wobei die Fühlerelemente (30, 31) eine Mehrzahl von Fühlerfingern (31) umfassen, die im Winkel gleichmäßig verteilt und dazu vorgesehen sind, sich in ausgefahrener Form in jeweils im spitzen Winkel zu einer Mitnahmelängsachse (62) geneigten Richtungen vor der Vorrichtung (10) zum Hohlraum (50, 52) hin zu erstrecken.

8. Implantationsvorrichtung (10) nach einem der Ansprüche 1 bis 7, wobei die Fühlerelemente (30, 31) fest mit verformbaren Mitteln (20) zum Aufnehmen eines Implantats (2) verbunden sind, die dazu vorgesehen sind, unter der Wirkung von Freigabemitteln (41) von einer eingefahrenen Form in eine radial ausgefahrene Form zum Anlegen an die Wand des rohrförmigen Elements (51) und zur Inbetriebnahme des Implantats (2) überzugehen.

9. Implantationsvorrichtung (10) nach einem der Ansprüche 1 bis 8, wobei die Mittel (20) zum Aufnehmen des Implantats (2) ein insgesamt zylinderförmiges Gitter aufweisen, um das Implantat zu tragen, das eine begrenzte Steifigkeit in radialer Richtung aufweist, und die Freigabemittel eine abnehmbare Hülle (41) zum Halten des Traggitters (20) in eingefahrener Form aufweisen, die sich axial über das Gitter hinaus erstreckt, um es durch relatives Zurücksetzen der Hülle (42) bezüglich eines Katheters (60) zum Verschieben der Vorrichtung freizugeben, wobei die Hemm- und Haltehüllen (41, 42) konzentrisch verlaufen.

10. Implantationsvorrichtung (10) nach einem der Ansprüche 1 bis 9, wobei die Mittel (20) zum Aufnehmen eines Implantats mit einem abdichtenden Seitenmantel (21) überdeckt sind, der dazu bestimmt ist, von den Mitteln (20) zum Aufnehmen eines Implantats an die Wand des rohrförmigen Elements (51) gedrückt zu werden, und der Mantel (21) wenigstens eine Öffnung oder Aussparung (22) aufweist, die eine bestimmte Winkelstellung zu den Fühlerelementen (30, 31) einnimmt.

11. Implantationsvorrichtung (10) nach einem der Ansprüche 1 bis 10, wobei die Fühlerelemente (131) ein zylindrisches Element aufweisen, das dazu vorgesehen ist, von einer eingefahrenen Form in eine radial ausgefahrene Form zum Abstützen an einer Wand des Hohlraums (52) unter der Wirkung von Teleaktivierungsmitteln überzugehen.

12. Implantationsvorrichtung (10) nach einem der Ansprüche 1 bis 11, wobei die Vorrichtung ein, zwei oder drei Fühlerelemente (30, 31) umfasst.

## Claims

1. Implantation device (10) for a valve comprising:
(a) a carrier element (20) configured to carry artificial valves (2) and configured to be able to be deployed from a collapsed configuration to a deployed configuration; and
(b) a catheter assembly (3, 41, 42, 60) configured to hold the implantation device (10) in a collapsed configuration when moving to a position upstream of the final targeted position;
**characterised in that** the implantation device (10) further comprises:
(c) at least one feeler element (30, 31) extending from the carrier element, the carrier element (20) and the feeler elements (30, 31) being disposed so that natural valves (50) can be pinched between the carrier element (20) and the feeler elements (30, 31);
while said at least one feeler element (30, 31) is deformable and is arranged to change from a collapsed form to an operational deployed form in order to detect a cavity (50, 52) and to position itself therein with reference to the position thereof.

2. Implantation device (10) according to claim 1, wherein the implantation device (10) is made up of a shape memory material and wherein the implantation device (10) is configured to be successively released from the catheter assembly (3, 41, 42, 60) so that at least one feeler element (30, 31) is released into a configuration that allows it to be received in a cavity (50, 52) associated with a natural valve (50) at a distant location, while the remainder of the implantation device (10) is kept in the collapsed configuration by the catheter assembly (3, 41, 42, 60).

3. Implantation device (10) according to claim 1 or 2, wherein the implantation device (10) can be fully released from the catheter assembly (3, 41, 42, 60) in a subsequent step.

4. Implantation device (10) according to any one of claims 1 to 3, wherein the feeler elements are based on a shape memory material.

5. Implantation device (10) according to any one of claims 1 to 4, wherein the catheter assembly (3, 41, 42, 60) is detachable and comprises a restriction sheath (42) for keeping the feeler elements (30, 31) in a collapsed position, which sheath extends beyond the feeler elements (30, 31) in order to release them through the relative backwards movement of the sheath relative to a catheter (60) for pushing the device.

6. Implantation device (10) according to any one of claims 1 to 5, wherein the feeler elements (30, 31) comprise a ring (30), of overall cylindrical shape, of wire loops (31) with limited rigidity in the radial direction, at least one of the loops (31) being provided in order to protrude laterally so as to form a feeler element.

7. Implantation device (10) according to any one of claims 1 to 6, wherein the feeler elements (30, 31) comprise a plurality of feeler fingers (31) that are evenly angularly distributed and are arranged, in the deployed form, to extend in respective inclined directions, at an acute angle, on a longitudinal axis (62) for forward-driving the device (10) towards the cavity (50, 52).

8. Implantation device (10) according to any one of claims 1 to 7, wherein the feeler elements (30, 31) are integral with deformable means (20) for receiving an implant (2) that are arranged, under the action of release means (41), to change from a collapsed form to a radially deployed form for bearing against the wall of the tubular element (51) and for activating the implant (2).

9. Implantation device (10) according to any one of claims 1 to 8, wherein the means (20) for receiving the implant (2) comprise a mesh of overall cylindrical shape for carrying the implant, having limited rigidity in the radial direction, and the release means comprise a detachable sheath (41) for keeping the carrier mesh (20) in the collapsed form, extending axially beyond the mesh in order to release it by the relative backwards movement of the sheath (42) relative to a catheter (60) for pushing the device, with the retention and restriction sheaths (41, 42) being concentric.

10. Implantation device (10) according to any one of claims 1 to 9, wherein the means (20) for receiving an implant are covered with a lateral sealing casing (21) intended to be pressed against the wall of the tubular element (51) by said means (20) for receiving an implant, and the casing (21) comprises at least one opening or indentation (22) occupying a determined angular position relative to the feeler elements (30, 31).

11. Implantation device (10) according to any one of claims 1 to 10, wherein the feeler means (131) comprise a cylindrical element arranged to change from a collapsed form to a radially deployed form for bearing against a wall of the cavity (52) under the effect of remote activation means.

12. Implantation device (10) according to any one of claims 1 to 11, wherein the device comprises one, two or three feeler elements (30, 31).
